# EUROPÄISCHE PATENTANMELDUNG

(11) **EP 4 819 093 A1**
(43) Veröffentlichungstag der Anmeldung: **07.10.2026**
(21) Anmeldenummer: 25167263.0
(22) Anmeldetag: 31.03.2025
(51) Int. Cl.: G08G 1/01, G06V 20/58, G08G 1/04, A61B 5/00, G08G 1/00, H04W 4/90, B60W 60/00, G08B 21/02, G08B 21/04, G08B 25/01

(54) **VERFAHREN UND FAHRZEUG ZUM ERKENNEN EINER NOTSITUATION EINER PERSON IM UMFELD DES FAHRZEUGES**

(71) Anmelder: Volkswagen Aktiengesellschaft, 38440 Wolfsburg (DE)
(72) Erfinder: Bader, Viktor, 38440 Wolfsburg (DE); Meinecke, Marc-Michael, 38440 Wolfsburg (DE)

(57) **Zusammenfassung**

Die Erfindung betrifft ein Verfahren zum Erkennen einer, bspw. medizinischen, Notsituation (N) einer Person (P) in einem Umfeld eines automatisiert und/oder autonom fahrenden Fahrzeuges (F), aufweisend:
- Erfassen von optischen und/oder akustischen Umfelddaten (D1, D2) für einen vorgegebenen Zeitraum (dt) durch mindestens eine Kameravorrichtung (K) und/oder mindestens ein Außenmikrofon (M) des Fahrzeuges (F),
- Auswerten von optischen und/oder akustischen Umfelddaten (D1, D2) durch eine Auswertevorrichtung (ECU) des Fahrzeuges (F), um eine Wahrscheinlichkeit (W) für die Notsituation (N) der Person (P) zu bestimmen,
- Vergleichen der Wahrscheinlichkeit (W) mit einem ersten Schwellenwert (W1) und einem zweiten Schwellenwert (W2) durch die Auswertevorrichtung (ECU), wobei der zweite Schwellenwert (W2) größer als der erste Schwellenwert (W1) ist,
- Erkennen der Notsituation (N) der Person (P) durch die Auswertevorrichtung (ECU) in Abhängigkeit von dem Vergleichen.

## Beschreibung

Die Erfindung betrifft ein Verfahren zum Erkennen einer, insbesondere medizinischen, Notsituation einer Person in einem Umfeld eines automatisiert und/oder autonom fahrenden Fahrzeuges sowie ein korrespondierendes, automatisiert und/oder autonom fahrendes Fahrzeug.

Autonom bzw. teilautonom fahrende Fahrzeuge sind grundsätzlich in der Lage, die Umgebung zu erfassen, zu analysieren und mithilfe von Umfelddaten selbstständig zu navigieren. Beispielhafte Sensorvorrichtungen sind bspw. Radar-, Lidar- und Bildsensoren. Damit autonom bzw. zumindest teilautonom fahrende Fahrzeuge (Level 4 bzw. 5) gesellschaftlich immer mehr akzeptiert werden, sollten sie möglichst nicht als Hindernis im Alltag gesehen werden, sondern sich bestenfalls ähnlich verhalten, wie es aufmerksame und hilfsbereite Fahrzeugführer machen würden. Beispielsweise wäre es ein großes Akzeptanzproblem für autonom bzw. teilautonom fahrende Fahrzeuge, wenn derartige Fahrzeuge in Not geratene Personen am Straßenrand nicht erkennen oder ignorieren, wohingegen aufmerksame und hilfsbereite Fahrzeugführer reagieren und helfen würden.

Autonom oder teilautonom fahrende Fahrzeuge sollten Notsituationen oder Situationen mit Gefährdungspotential für Personen im Umfeld des Fahrzeugs mit der Auswertung von Umfeldsensoren erkennen, interpretieren und ggf. Hilfemaßnahmen selbstständig ausführen und/oder einleiten können. Zugleich sollen häufige Fehlalarme für z. B. das Auslösen von Notrufen vermieden werden. Daher wird ein potentieller Notfall meistens nur dann erkannt, wenn eine hohe Wahrscheinlichkeit durch die Auswertung von Umfelddaten vorliegt.

In neueren Fahrzeugen wird eine Zusatzabfrage vorgeschlagen, um zu ermitteln, ob eine Notsituation gegeben ist. Die Zusatzabfrage kann an einen Insassen des Fahrzeugs gerichtet werden, der zu einer Eingabe aufgefordert wird. Durch die Zusatzabfrage wird der Insasse auf die wahrscheinliche Notsituation aufmerksam gemacht, kann das Umfeld des Fahrzeuges prüfen und das Vorliegen der Notsituation gegenüber dem Fahrzeug bestätigen oder umgekehrt angeben, dass die Situation nicht kritisch ist. Alternativ kann die Zusatzabfrage an Personen außerhalb des Kraftfahrzeugs gerichtet werden

Nachteilig kann die Zusatzabfrage insbesondere dann sein, wenn das autonom fahrende Fahrzeug ohne Fahrzeuginsassen unterwegs ist oder wenn keine Personen zum Befragen verfügbar sind.

Eine beispielhafte Schrift zum allgemeinen Stand der Technik ist die DE 10 2029 211 591 B4.

Es ist daher Aufgabe der vorliegenden Erfindung, wenigstens einen der voranstehend beschriebenen Nachteile zumindest teilweise zu überwinden. Insbesondere ist es Aufgabe der Erfindung, ein verbessertes Verfahren zum (vorzugsweise automatischen) Erkennen einer, bspw. medizinischen, Notsituation einer Person in einem Umfeld eines automatisiert und/oder autonom fahrenden Fahrzeuges (bevorzugt ohne eine Zusatzabfrage eines Benutzers des Fahrzeuges bzw. ohne eine Freigabe durch einen Benutzer) zur Verfügung zu stellen. Vorzugsweise ist es Aufgabe der Erfindung, ein solches Verfahren bereitzustellen, welches ein schnelles, sicheres und zuverlässiges Erkennen von Notsituationen oder Situationen mit Gefährdungspotential für Personen im Umfeld des Fahrzeugs ermöglicht, welches Fehlalarme zuverlässig vermeidet, welches die Sicherheit für Personen im Umfeld des Fahrzeugs erhöht, welches die Akzeptanz für die automatisiert und/oder autonom fahrenden Fahrzeuge steigert und welches es ermöglicht, dass die automatisiert und/oder autonom fahrenden Fahrzeuge wie aufmerksame und hilfsbereite Fahrzeugführer reagieren und sogar helfen können. Bevorzugt ist es Aufgabe der Erfindung, ein solches Verfahren bereitzustellen, welches zusätzliche Abfragen von Benutzern, wie z. B. Fahrzeuginsassen, vermeidet.

Die voranstehende Aufgabe wird gelöst durch ein Verfahren gemäß einem ersten Aspekt der vorliegenden Erfindung sowie durch ein Fahrzeug gemäß einem zweiten Aspekt der vorliegenden Erfindung.

Weitere Merkmale und Details der Erfindung ergeben sich aus den Unteransprüchen, der Beschreibung und den Zeichnungen. Dabei gelten Merkmale und Details, die im Zusammenhang mit dem erfindungsgemäßen Verfahren beschrieben sind, selbstverständlich auch im Zusammenhang mit dem erfindungsgemäßen Fahrzeug und jeweils umgekehrt, sodass bezüglich der Offenbarung zu den einzelnen Erfindungsaspekten stets wechselseitig Bezug genommen wird bzw. werden kann.

Gemäß einem ersten Aspekt betrifft die vorliegende Erfindung ein Verfahren zum (vorzugsweise automatischen) Erkennen einer, bspw. medizinischen, Notsituation einer Person (oder mehrerer Personen) in einem Umfeld eines automatisiert und/oder autonom fahrenden Fahrzeuges (bevorzugt ohne eine Zusatzabfrage einer Person innerhalb oder außerhalb des Fahrzeuges).

Das Verfahren weist folgende Verfahrensschritte auf:
- Erfassen (oder mit anderen Worten Sensieren) von optischen und/oder akustischen Umfelddaten für einen vorgegebenen (insbesondere gezielt ausgewählten) Zeitraum durch mindestens eine Kameravorrichtung und/oder mindestens ein Außenmikrofon des Fahrzeuges,
- Auswerten von optischen und/oder akustischen Umfelddaten durch eine Auswertevorrichtung des Fahrzeuges,
   - um eine Wahrscheinlichkeit für die Notsituation der Person(en) zu bestimmen,
   - und insbesondere die Notsituation zu klassifizieren,
   - und vorzugsweise ein Gefährdungspotential für die Person(en) in der Notsituation, bevorzugt in Graden, zu bestimmen,
- Vergleichen der Wahrscheinlichkeit mit einem ersten Schwellenwert und einem zweiten Schwellenwert durch die Auswertevorrichtung, wobei der zweite Schwellenwert größer als der erste Schwellenwert ist,
- Erkennen der Notsituation der Person durch die Auswertevorrichtung in Abhängigkeit von dem Vergleichen.

Die Notsituation kann ebenfalls als ein Notfall oder ein Unfall bezeichnet werden. Die Notsituation kann eine oder mehrere Personen betreffen.

Das Verfahren kann vorzugsweise wiederholend im laufenden Betrieb des Fahrzeuges durchgeführt werden.

Vorteilhaft kann es dabei sein, dass ein autonom bzw. teilautonom fahrendes Fahrzeug in seinem Umfeld (auch neben der Fahrbahn und ggf. auch beim Parken) möglichst Gefahren- bzw. Notsituationen von Person(en) (eigenständig) erkennt, interpretiert und ggf. dann sinnvoll und gefahrenminimierend reagiert (z. B. eigenständig ein Notfallsignal an eine Notfallzentrale sendet oder - soweit möglich - eine Unfallstelle markiert und absichert).

Es gibt verschiedene Arten von Notsituationen im Verkehr, die Personen betreffen können und die mithilfe des Verfahrens klassifiziert werden können, bspw.:
1. Verkehrsunfälle: Kollisionen zwischen Fahrzeugen oder zwischen Fahrzeugen und Fußgängern, die zu Verletzungen oder sogar Todesfällen führen können.
2. Medizinische Notfälle: Personen, die während der Fahrt plötzlich gesundheitliche Probleme wie Herzinfarkte oder Schlaganfälle erleiden.
3. Pannen: Fahrzeugausfälle, die dazu führen, dass Personen in einer gefährlichen Situation auf der Straße stehen bleiben müssen.
4. Übergriffe oder Gewalt: Situationen, in denen Personen im Verkehr Opfer von Übergriffen oder Gewalt werden.
5. Naturkatastrophen: Ereignisse wie Überschwemmungen, Erdbeben oder Stürme, die den Verkehr gefährden und Personen in Gefahr bringen können.
6. Fahrzeugbrände: Brände, die in Fahrzeugen ausbrechen und die Insassen in Gefahr bringen.

Es ist wichtig, in solchen Situationen schnell und angemessen zu handeln, um die Sicherheit aller Beteiligten zu gewährleisten.

Das Gefährdungspotential kann in verschiedene Grade eingeteilt werden, um die Schwere einer Notsituation besser einschätzen zu können, die mithilfe des Verfahrens bestimmt werden können, bspw.:
1. Geringes Gefährdungspotential: Situationen, die wenig bis keine Gefahr für Personen darstellen.
2. Mäßiges Gefährdungspotential: Situationen, die ein gewisses Risiko für Personen darstellen, wenn die Personen z. B. am Straßenrand stehen.
3. Hohes Gefährdungspotential: Situationen, die ernsthafte Gefahren für Personen darstellen, wie z. B. ein schwerer Verkehrsunfall mit Verletzten oder ein Fahrzeugbrand, der schnell eskalieren kann.
4. Extremes Gefährdungspotential: Lebensbedrohliche Situationen, wie z. B. ein Unfall mit mehreren Fahrzeugen, bei dem viele Personen verletzt sind, oder Naturkatastrophen.

Das Verfahren kann vorteilhafterweise dazu dienen, solche Notsituationen für Personen nicht nur schnell, sicher und zuverlässig zu erkennen, sondern auch zu klassifizieren und sogar das Gefährdungspotential zu bestimmen. Mithilfe der Klassifikation und des Gefährdungspotentials können zielgerichtet passende Maßnahmen eingeleitet werden, um die Notsituation auf eine verbesserte Weise zu beheben und/oder das Gefährdungspotential möglichst unverzüglich zu eliminieren.

Zum Durchführen des Verfahrens können zunächst Umfeldkamera-Daten (mit Objekt-, Gesten- und/oder Bewegungserkennung) und/oder Umfeld-Akustikdaten (mit Geräusch- und/oder Spracherkennung) von Mikrofonen des Fahrzeugs erfasst und ausgewertet werden.

Ein potentieller Notfall und/oder eine potentielle Gefährdungssituation von einer Person im Fahrzeugumfeld kann erkannt werden, wenn die Wahrscheinlichkeit für die Notsituation oberhalb des ersten Schwellwertes liegt.

Danach kann die Wahrscheinlichkeit für die Notsituation mit dem zweiten Schwellenwert verglichen werden.

Bspw. ist es denkbar, dass die Wahrscheinlichkeit für den weiteren Vergleich mit dem zweiten Schwellenwert mithilfe von Umfelddaten weiterer Sensoren angepasst werden kann, wie z. B. mithilfe von Umfelddaten eines, vorzugsweise hochauflösenden, Fahrzeugradarsystems, welches bevorzugt unabhängig vom Umfeldkamerasystem und von Fahrzeugmikrofonen Umfelddaten erfasst und auswertet. Die Umfelddaten weiterer Sensoren können vorteilhafterweise bezüglich derselben Person(en) ausgewertet werden, die mithilfe von optischen und/oder akustischen Umfelddaten als gefährdete Person(en) in der Notsituation erkannt wurde(n). Dabei können vorteilhafterweise zugehörige Biovitaldaten erkannt werden, wie z. B. Atem- und/oder Herzfrequenz usw. Wenn nun die angepasste Wahrscheinlichkeit oberhalb des zweiten Schwellenwerts liegt, kann die Notfallsituation nahezu sicher erkannt werden. Ferner kann die Notsituation klassifiziert und/oder Gefährdungspotential für die betroffene(n) Person(en) bestimmt werden, sodass passende Notfallmaßnahmen eingeleitet werden können.

Außerdem ist es denkbar, dass bereits nach dem Auswerten von optischen und/oder akustischen Umfelddaten die Wahrscheinlichkeit für die Notsituation bereits über dem zweiten Schwellenwert liegen kann, wenn bspw. anhand von Bilddaten relativ sicher schwere Unfälle mit hohem Gefährdungspotential erkannt werden und/oder wenn bspw. anhand von akustischen Daten relativ sicher Hilferufe erkannt werden. Auf diese Weise können Hilfemaßnahmen besonders zeitnahe und ohne Zeitverzögerungen eingeleitet werden.

Mithilfe der Auswertung von radarbasierten Umfelddaten kann die Notfallsituation genauer klassifiziert und/oder das Gefährdungspotential eindeutiger bestimmt werden, sodass passende Notfallmaßnahmen zielgerichtet eingeleitet werden können.

Mithilfe der Auswertung von optischen und/oder akustischen Umfelddaten kann ferner eine Verortung einer Person erfolgen. Durch eine Verortung derselben Person mithilfe von Radarsensoren können ermittelte Biovitaldaten der betroffenen Person zugeordnet werden. Mithilfe von Vitaldaten können präzisere Notfallmaßnahmen festgelegt oder ggf. eine besondere Dringlichkeit für zeitnahe Ausführung von Maßnahmen festgestellt und umgesetzt werden.

Mithilfe der Auswertung von radarbasierten Umfelddaten können sogar nicht sichtbare, sich z. B. hinter einem Buschwerk befindende, Person(en) erkannt werden. Dabei können akustische Umfelddaten - Spricht oder ruft die nicht sichtbare Person? - dazu dienen, die Wahrscheinlichkeit für die Notsituation mit dem ersten Schwellenwert zu vergleichen. Die radarbasierten Umfelddaten können dabei dazu dienen, die Wahrscheinlichkeit für die Notsituation zu verifizieren bzw. die angepasste Wahrscheinlichkeit mit dem zweiten Schwellenwert zu vergleichen. Können Radarreflexionssignale der gleichen Person zugeordnet werden und übersteigt die angepasste Wahrscheinlichkeit den zweiten Schwellenwert, dann liegt sehr wahrscheinlich eine Notsituation vor.

Zum Erfassen und Auswerten von radarbasierten Umfelddaten kann vorzugsweise ein Radarsystem mit mehreren Radarsensoren verwendet werden, die um das Fahrzeug herum angeordnet werden können.

Mithilfe eines solchen Radarsystems ist es möglich, für Distanzen über 50 m hinaus (und sogar z. B. durch ein Buschwerk hindurch) Personen zu verorten und auf ihre Atem- und Herzfrequenz zu schließen, um damit auf ihren Gesundheitszustand zu schließen und ggf. einen gesundheitlichen Notfall zu erkennen bzw. den Verdacht abzusichern.

Eine beispielhafte Ausführungsform kann folgende Details vorsehen:
1. Mithilfe von Umfeldkamera-Aufnahmen und/oder Außen-Mikrofon-Aufnahmen und einer Auswerteeinrichtung für Bilddaten und/oder akustische Signaldaten werden Daten für einen vorgegebenen zurückliegenden Zeitraum ausgewertet und dahingehend überprüft, ob ein erster Schwellenwert für eine Wahrscheinlichkeit für eine (klassifizierbare) Notsituation und/oder ein Gefährdungspotential einer (verorteten) Person im Fahrzeugumfeld überschritten ist.
2. Wird in Schritt 1 eine Wahrscheinlichkeit für eine (klassifizierte) Notsituation und/oder ein Gefährdungspotential aus den Bilddaten und/oder akustischen Daten erkannt, die über dem Schwellwert liegt, wird für einen vorgegebenen zurückliegenden Zeitraum und/oder aktuellen Zeitpunkt überprüft, ob Radarreflexionssignale der verorteten Person genügend wahrscheinlich zugeordnet und ausgewertet werden können, die genügend wahrscheinlich auf Muster der Atem- und/oder Herzfrequenz als Biovitaldaten der verorteten Person schließen lassen.
3. Liegen genügend gute Daten über die Atem- und/oder Herzfrequenzmuster bezüglich der verorteten Person aus Schritt 2 vor, wird unter Einbezug dieser Daten eine neue Wahrscheinlichkeit für die medizinische Notsituation und/oder das Gefährdungspotential dieser (verorteten) Person ermittelt.
4. Liegt die angepasste Wahrscheinlichkeit aus Schritt 3 über einem (gegenüber dem ersten Schwellenwert höheren) vorgegebenen zweiten Schwellwert für eine medizinische Notsituation oder einem Gefährdungspotential der verorteten Person, so werden seitens des Fahrzeugs je nach erkannter (klassifizierter) medizinischer Notsituation und/oder je nach (bestimmtem) Gefährdungspotential, erkanntem Kontext und Umgebungsbedingungen, Maßnahmen eingeleitet, wie z. B. Aussenden eines Notfallsignals, vorzugsweise mit verfügbaren Informationen, Markieren und/oder Absichern des Unfallortes und/oder der verorteten Person usw.

Bei Schritt 2 kann ggf. bereits alleine aus den Bilddaten und/oder akustischen Daten darauf geschlossen werden, dass eine Wahrscheinlichkeit für eine Notsituation und/oder ein Gefährdungspotential der Person vorliegt, die bereits oberhalb des zweiten Schwellwertes liegt. So können auch ohne das Vorliegen bzw. Auswerten von radarbasierten Umfelddaten Notfallmaßnahmen durchgeführt und/oder eingeleitet werden.

Vorteilhafterweise können auch in dem letzteren Falle die radarbasierten Umfelddaten genutzt werden, um daraus Vitalparameter, wie z. B. Atem- und/ oder Herzfrequenzmuster der betroffenen Person, zu ermitteln. Diese Vitalparameter können bspw. einer Notfallmeldung beigefügt werden.

Zusätzlich kann überprüft werden, ob weitere Personen sich in der unmittelbaren Umgebung der betroffenen Person befinden, die sich bereits um diese kümmern. Dann brauchen keine weiteren Hilfemaßnahmen seitens des Fahrzeugs eingeleitet werden.

Vorteilhafterweise kann das Fahrzeug zur Bewertung der Notsituation und/oder zur verbesserten Klassifizierung der erkannten Notsituation dazu veranlasst werden, sich gegenüber der Person möglichst optimal zu positionieren, sodass möglichst optimal Vitalparameter ermittelt werden können.

Außerdem ist es denkbar, dass, wenn von einem ersten autonomen Fahrzeug genügend wahrscheinlich eine Notfallsituation bei einer Person erkannt wurde, ein zweites autonomes Fahrzeug aus einer Fahrzeugflotte mit präziseren Umfeldsensoren (z. B. mit einem hochauflösenden COALA-Fahrzeugradarsystem) in einem (z. B. entsprechend einer ersten Klassifizierung der Unfallsituation) räumlichen oder zeitlichen Umkreis zu der Unfallstelle geschickt wird. Das zweite Fahrzeug wird dazu veranlasst, sich zu der Unfallstelle zu bewegen und Vitalparameter der Person in der Notsituation zu ermitteln. Hierzu kann das erste Fahrzeug die Notfallsituation an eine zentrale Backendeinheit melden, die das zweite Fahrzeug schicken kann. Zudem kann das erste Fahrzeug auch über V2V-Kommunikation die Notfallsituation umliegenden Fahrzeugen melden, die die Daten der Backendeinheit oder direkt einem passenden zweiten Fahrzeug weiterleiten können.

Zum einem kann vorgesehen sein, dass, wenn die Wahrscheinlichkeit für eine Notsituation anhand von optischen und/oder akustischen Umfelddaten über dem ersten Schwellwert und unter dem zweiten Schwellenwert liegt, das Verfahren mindestens einen von den folgenden Verfahrensschritten umfassen kann:
- Erfassen von radarbasierten Umfelddaten für den vorgegebenen Zeitraum,
- Überprüfen, ob die radarbasierten Umfelddaten der Person in der Notsituation zugeordnet werden können, durch die Auswertevorrichtung,
- Auswerten von radarbasierten Umfelddaten, die der Person in der Notsituation zugeordnet werden können, durch die Auswertevorrichtung, um Vitalparameter, bspw. Atem- und/oder Herzfrequenz der Person in der erkannten Notsituation zu bestimmen,
- Anpassen der Wahrscheinlichkeit für die Notsituation in Abhängigkeit von den Vitalparametern durch die Auswertevorrichtung,
- Vergleichen der angepassten Wahrscheinlichkeit mit dem zweiten Schwellenwert und/oder
- Erkennen der Notsituation mithilfe von radarbasierten Umfelddaten durch die Auswertevorrichtung, wenn die angepasste Wahrscheinlichkeit über dem zweiten Schwellenwert liegt.

Zum anderen kann vorgesehen sein, dass, wenn die Wahrscheinlichkeit für eine Notsituation anhand von optischen und/oder akustischen Umfelddaten über dem zweiten Schwellenwert liegt, ferner aufweisend:
- Erkennen der Notsituation mithilfe von optischen und/oder akustischen Umfelddaten, insbesondere ohne radarbasierte Umfelddaten oder bevor radarbasiere Umfelddaten erfasst und ausgewertet werden.

Vorteilhafte Hilfemaßnahmen können bspw. sein:
- Aussenden einer Notfallmeldung an Rettungskräfte, an einen Erste-Hilfe-Ort (bspw. Krankenhaus, nächstliegende Arztpraxis usw.), an andere Fahrzeuge, an Infrastruktur und/oder an eine Backendvorrichtung,
- Anhalten des Fahrzeuges,
- Markieren und/oder Absichern eines Notfallortes,
- Bereitstellen einer Transportmöglichkeit für die Person zu einem Erste-Hilfe-Ort,
- Übermitteln von Klassifikationsdaten für die Notsituation an die Backendvorrichtung, an Rettungskräfte und/oder einen Erste-Hilfe-Ort,
- Übermitteln von Vitalparametern an die Backendvorrichtung, an Rettungskräfte und/oder einen Erste-Hilfe-Ort und/oder
- Übermitteln von optischen, akustischen und/oder radarbasierten Umfelddaten an die Backendvorrichtung, an Rettungskräfte und/oder an einen Erste-Hilfe-Ort.

Es ist ferner denkbar, dass der vorgegebene Zeitraum und/oder die Schwellenwerte in Abhängigkeit von mindestens einem von den folgenden Parametern bestimmt wird/werden:
- Ort,
- Verkehrssituation,
- Kontextsituation, z. B. erkannte Menschenansammlung unmittelbar bei der Person in der Notsituation,
- Umgebungsbedingungen,
- Wetterbedingungen,
- Tageszeit,
- Jahreszeit und/oder
- Wochentag.

In Abhängigkeit von diesen Parametern können situationsgemäß der vorgegebene Zeitraum und/oder die Schwellenwerte bestimmt werden. Bspw. kann bei Tageslicht und wenig Verkehr der vorgegebene Zeitraum verlängert und/oder die Schwellenwerte angehoben werden. Bspw. kann bei schlechten Wetterbedingungen und/oder Lichtbedingungen der vorgegebene Zeitraum reduziert und/oder die Schwellenwerte herabgesetzt werden.

Ferner kann vorgesehen sein, dass die optischen Umfelddaten durch mindestens eine oder mehrere Außenkamera(s) erfasst werden. Aufnahmen von mehreren Außenkameras können dazu dienen, das Umfeld des Fahrzeuges möglichst vollumfassend zu erkennen. Die optischen Umfelddaten können vorteilhafterweise mithilfe einer Bildverarbeitung und/oder einer Bildsegmentierung, insbesondere einer semantischen Bildsegmentierung, einer Objekterkennung, einer Gestenerkennung und/oder einer Bewegungserkennung ausgewertet werden. Auf diese Weise kann eine mögliche Notsituation bereits genügend wahrscheinlich mit Bilddaten erkannt werden. Bspw. kann eine am Straßenrand liegende Person, die in den Bilddaten erkannt wird, ziemlich wahrscheinlich auf eine potentielle Notsituation hinweisen.

Weiterhin kann vorgesehen sein, dass die akustischen Umfelddaten durch ein Stereosystem mit mindestens zwei oder mehreren Außenmikrofonen erfasst werden, um insbesondere eine Geräuschlokalisierung zu ermöglichen. Die akustischen Umfelddaten können vorteilhafterweise mithilfe einer Geräuscherkennung, einer Geräuschlokalisierung und/oder einer Spracherkennung ausgewertet werden. Auf diese Weise können weitere Indizien gefunden werden, die ziemlich genau auf eine potentielle Notsituation hinweisen können, wie z. B. Ausrufesätze, Hilferufe usw.

Des Weiteren kann vorgesehen sein, dass die radarbasierten Umfelddaten durch ein Radarsystem mit mindestens zwei oder mehreren Radarsensoren erfasst werden, um insbesondere eine hochaufgelöste Radaranalyse zu ermöglichen. Das Radarsystem kann dabei die radarbasierten Umfelddaten von mehreren Radarsensoren kombinieren, um das Umfeld des Fahrzeuges möglichst vollumfassend zu erkennen. Im Rahmen der Erfindung kann es von Vorteil sein, dass die radarbasierten Umfelddaten mithilfe einer FMCW-Radartechnologie oder einer UWB-Radartechnologie erfasst und ausgewertet werden. Auf diese Weise können hochaufgelöste Daten ermittelt werden, die auf die Position, Geschwindigkeit, Winkel und/oder deren zeitliche Veränderung hindeuten. Auf diese Weise können Vitalparameter einer Person in der Not bestimmt werden.

Grundsätzlich ist es denkbar, dass das Verfahren durch ein Rettungsfahrzeug ausgeführt wird. Vorteilhafterweise kann ferner vorgesehen sein, dass, wenn sich das Rettungsfahrzeug einer Unfallstelle nähert, die optischen, akustischen und/oder radarbasierte Umfelddaten und/oder Vitalparameter der Person in der Notsituation und/oder Klassifikationsdaten für die Notsituation und/oder bestimmtes Gefährdungspotential den Rettungskräften, Notfallsanitätern und/oder Notfallärzten im Rettungsfahrzeug aufgezeigt und/oder zur Bewertung und/oder Veranlassung von weiteren Maßnahmen an einen Erste-Hilfe-Ort und/oder an eine Backendvorrichtung gesendet werden. Auf diese Weise kann eine verbesserte Kette von helfenden Maßnahmen ermöglicht werden.

Es kann im Rahmen der Erfindung vorgesehen sein, dass das Fahrzeug zum Durchführen des Verfahrens sich gegenüber der Person in der Notsituation positioniert, um Vitalparameter der Person auf eine verbesserte Weise bestimmen zu können. Somit kann die Notsituation nicht nur sicher erkannt, sondern auch auf eine verbesserte Weise klassifiziert und/oder beschrieben werden.

Es ist ferner denkbar, dass, wenn eine Notsituation erkannt wurde, ein spezielles Fahrzeug mit hochaufgelösten Umfeldsensoren geschickt wird (bspw. durch das Fahrzeug selbst und/oder mithilfe einer Backendvorrichtung, die durch das Fahrzeug über die Notsituation informiert wurde), um die Notsituation zu verifizieren und/oder mindestens eine weitere Hilfemaßnahme bereitzustellen. Somit kann sichergestellt werden, dass die Notsituation möglichst genau analysiert wird.

Gemäß einem zweiten Aspekt betrifft die vorliegende Erfindung ferner ein Fahrzeug zum Durchführen eines Verfahrens zum Erkennen einer, bspw. medizinischen, Notsituation einer Person in einem Umfeld eines automatisiert und/oder autonom fahrenden Fahrzeuges, welches wie oben beschrieben ablaufen kann,
aufweisend:
- mindestens eine Kameravorrichtung und/oder mindestens ein Außenmikrofon und/oder mindestens einen Radarsensor zum Erfassen von optischen, akustischen und/oder radarbasierten Umfelddaten für einen vorgegebenen Zeitraum,
- eine Auswertevorrichtung zum Auswerten von optischen, akustischen und/oder radarbasierten Umfelddaten, die dazu ausgeführt ist,
   - um eine Wahrscheinlichkeit für die Notsituation der Person(en) zu bestimmen,
   - und insbesondere die Notsituation zu klassifizieren,
   - und vorzugsweise ein Gefährdungspotential für die Person(en) in der Notsituation, bevorzugt in Graden, zu bestimmen,

wobei die Auswertevorrichtung zum Vergleichen der Wahrscheinlichkeit mit einem ersten Schwellenwert und einem zweiten Schwellenwert ausgeführt ist,
wobei die Auswertevorrichtung zum Erkennen der Notsituation in Abhängigkeit von dem Vergleichen ausgeführt ist,
wobei insbesondere die Auswertevorrichtung zum Veranlassen und/oder Durchführen mindestens einer Hilfemaßnahme ausgeführt ist,
wobei vorzugsweise die Auswertevorrichtung zum Ansteuern von der mindestens einen Kameravorrichtung und/oder dem mindestens einen Außenmikrofon und/oder dem mindestens einen Radarsensor zum Erfassen von optischen, akustischen und/oder radarbasierten Umfelddaten für einen vorgegebenen Zeitraum ausgeführt ist.

Damit ergeben sich in Bezug auf ein erfindungsgemäßes Fahrzeug die gleichen Vorteile, wie sie bereits in Bezug auf ein erfindungsgemäßes Verfahren beschrieben worden sind.

Weitere Vorteile, Merkmale und Einzelheiten der Erfindung ergeben sich aus der nachfolgenden Beschreibung, in der unter Bezugnahme auf die Zeichnungen mehrere Ausführungsbeispiele der Erfindung im Einzelnen beschrieben sind. Dabei können die in den Ansprüchen und in der Beschreibung erwähnten Merkmale jeweils einzeln für sich oder in beliebiger Kombination erfindungswesentlich sein. Dabei zeigt schematisch:

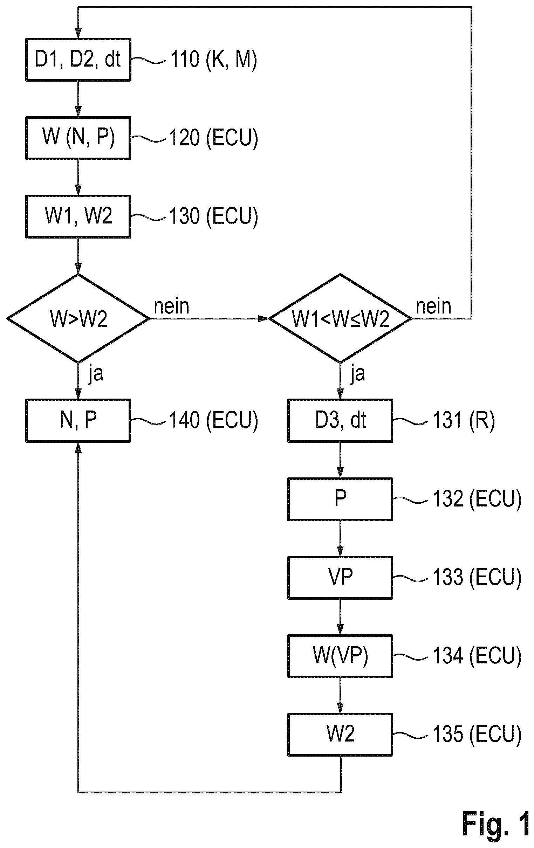

- Figur 1: einen beispielhaften Ablauf eines vorgeschlagenen Verfahrens.

Die vorliegende Erfindung betrifft ein Verfahren zum (vorzugsweise automatischen, fahrzeugeigenständigen und vorzugsweise ohne menschliche Hilfe) Erkennen einer, bspw. medizinischen, Notsituation N einer Person P (oder mehrerer Personen) in einem Umfeld eines automatisiert und/oder autonom fahrenden Fahrzeuges F (im Weiteren einfach als Fahrzeug F bezeichnet).

Das Verfahren kann bevorzugt ohne eine Zusatzabfrage einer Person innerhalb oder außerhalb des Fahrzeuges durchgeführt werden.

Wie es die Fig. 1 andeutet, weist das Verfahren folgende Verfahrensschritte auf:
110 Erfassen (oder mit anderen Worten Erheben) von optischen und/oder akustischen Umfelddaten D1, D2 für einen vorgegebenen (insbesondere gezielt ausgewählten) Zeitraum dt durch mindestens eine Kameravorrichtung K und/oder mindestens einen Außenmikrofon M des Fahrzeuges F,
120 Auswerten von optischen und/oder akustischen Umfelddaten D1, D2 durch eine Auswertevorrichtung ECU des Fahrzeuges F,
   - um eine Wahrscheinlichkeit W für die Notsituation N der Person(en) P zu bestimmen,
   - und insbesondere die Notsituation N zu klassifizieren,
   - und vorzugsweise ein Gefährdungspotential für die Person(en) P in der Notsituation, bevorzugt in Graden, zu bestimmen,
130 Vergleichen der Wahrscheinlichkeit W mit einem ersten Schwellenwert W1 und einem zweiten Schwellenwert W2 durch die Auswertevorrichtung ECU, wobei der zweite Schwellenwert W2 größer als der erste Schwellenwert W1 ist,
140 Erkennen der Notsituation N der Person(en) P durch die Auswertevorrichtung ECU in Abhängigkeit von dem Vergleichen.

Die Auswertevorrichtung ECU kann vorteilhafterweise dazu ausgeführt sein, folgende Klassifikationen 1 bis 6 einer erkannten Notsituation zu bestimmen, bspw.:
1. Verkehrsunfälle,
2. medizinische Notfälle,
3. Pannen,
4. Übergriffe oder Gewalt,
5. Naturkatastrophen und/oder
6. Fahrzeugbrände.

Die Auswertevorrichtung ECU kann vorteilhafterweise dazu ausgeführt sein, folgende Grade 1 bis 4 bei dem Gefährdungspotential zu bestimmen, bspw.:
1. Geringes Gefährdungspotential,
2. mäßiges Gefährdungspotential,
3. hohes Gefährdungspotential, und/oder
4. extremes Gefährdungspotential.

Die Auswertevorrichtung ECU kann ferner zum Veranlassen und/oder Durchführen mindestens einer Hilfemaßnahme ausgeführt sein.

Vorzugsweise kann die Auswertevorrichtung ECU zum Ansteuern von der mindestens einen Kameravorrichtung K und/oder dem mindestens einen Außenmikrofon M und/oder dem mindestens einen Radarsensor R zum Erfassen von optischen, akustischen und/oder radarbasierten Umfelddaten D1, D2, D3 für einen vorgegebenen Zeitraum dt ausgeführt sein.

Das Verfahren kann vorteilhafterweise dazu dienen, Notsituationen N für Personen P nicht nur schnell, sicher und zuverlässig zu erkennen, sondern auch zu klassifizieren und sogar das Gefährdungspotential zu bestimmen. Mithilfe der Klassifikation und des Gefährdungspotentials können zielgerichtet passende Hilfemaßnahmen eingeleitet werden, um die Notsituation N auf eine verbesserte Weise zu beheben und/oder das Gefährdungspotential für Personen P zu reduzieren.

Zum einem kann vorgesehen sein, dass, wenn die Wahrscheinlichkeit W für eine Notsituation N anhand von optischen und/oder akustischen Umfelddaten über dem ersten Schwellwert W1 und unter dem zweiten Schwellenwert W2 liegt, das Verfahren mindestens einen von den folgenden Verfahrensschritten umfassen kann:
131 Erfassen von radarbasierten Umfelddaten D3 für den vorgegebenen Zeitraum dt,
132 Überprüfen durch die Auswertevorrichtung ECU, ob die radarbasierten Umfelddaten D3 der Person P in der Notsituation N zugeordnet werden können,
133 Auswerten von radarbasierten Umfelddaten D3, die der Person in der Notsituation N zugeordnet werden können, durch die Auswertevorrichtung ECU, um Vitalparameter VP, bspw. Atem- und/ oder Herzfrequenz, der Person P in der erkannten Notsituation N zu bestimmen,
134 Anpassen der Wahrscheinlichkeit W für die Notsituation N in Abhängigkeit von den Vitalparametern VP durch die Auswertevorrichtung ECU,
135 Vergleichen der angepassten Wahrscheinlichkeit W mit dem zweiten Schwellenwert W2 durch die Auswertevorrichtung ECU und/oder
140 Erkennen der Notsituation N mithilfe von radarbasierten Umfelddaten D3 durch die Auswertevorrichtung ECU, wenn die angepasste Wahrscheinlichkeit W über dem zweiten Schwellenwert W2 liegt.

Zum anderen kann vorgesehen sein, dass, wenn die Wahrscheinlichkeit W für eine Notsituation N anhand von optischen und/oder akustischen Umfelddaten D1, D2 über dem zweiten Schwellenwert W2 liegt, ferner aufweisend:
140 Erkennen der Notsituation N mithilfe von optischen und/oder akustischen Umfelddaten D1, D2 durch die Auswertevorrichtung ECU, insbesondere ohne radarbasierte Umfelddaten D3 oder bevor radarbasiere Umfelddaten D3 (für mehr Informationen über die Notsituation N und/oder über das Gefährdungspotential der Person P) erfasst und ausgewertet werden.

Mögliche Hilfemaßnahmen sind bspw.:
- Aussenden einer Notfallmeldung an Rettungskräfte, an einen Erste-Hilfe-Ort, bspw. Krankenhaus, nächstliegende Arztpraxis usw., an andere Fahrzeuge, an Infrastruktur und/oder an eine Backendvorrichtung,
- Anhalten des Fahrzeuges F,
- Markieren und/oder Absichern eines Notfallortes,
- Bereitstellen einer Transportmöglichkeit für die Person P zu einem Erste-Hilfe-Ort,
- Übermitteln von Klassifikationsdaten für die Notsituation N an die Backendvorrichtung, an Rettungskräfte und/oder einen Erste-Hilfe-Ort,
- Übermitteln von Vitalparametern VP an die Backendvorrichtung, an Rettungskräfte und/oder einen Erste-Hilfe-Ort und/oder
- Übermitteln von optischen, akustischen und/oder radarbasierten Umfelddaten D1, D2, D3 an die Backendvorrichtung, an Rettungskräfte und/oder an einen Erste-Hilfe-Ort.

Vorteilhafterweise kann/können der vorgegebene Zeitraum dt und/oder die Schwellenwerte W1, W2 in Abhängigkeit von mindestens einem von den folgenden Parametern bestimmt werden:
- Ort,
- Verkehrssituation,
- Kontextsituation, z. B. erkannte Menschenansammlung unmittelbar bei der Person P in der Notsituation N,
- Umgebungsbedingungen,
- Wetterbedingungen,
- Tageszeit,
- Jahreszeit und/oder
- Wochentag.

Die optischen Umfelddaten D1 können durch mindestens eine oder mehrere Außenkamera(s) erfasst und vorteilhafterweise mithilfe einer Bildverarbeitung und/oder einer Bildsegmentierung, insbesondere einer semantischen Bildsegmentierung, einer Objekterkennung, einer Gestenerkennung und/oder einer Bewegungserkennung ausgewertet werden.

Die akustischen Umfelddaten D2 können durch ein Stereosystem mit mindestens zwei oder mehreren Außenmikrofonen erfasst werden, um insbesondere eine Geräuschlokalisierung zu ermöglichen. Die akustischen Umfelddaten D2 können vorteilhafterweise mithilfe einer Geräuscherkennung, einer Geräuschlokalisierung und/oder einer Spracherkennung ausgewertet werden.

Die radarbasierten Umfelddaten D3 können durch ein Radarsystem mit mindestens zwei oder mehreren Radarsensoren R erfasst werden, um insbesondere eine hochaufgelöste Radaranalyse zu ermöglichen. Das Radarsystem kann dabei die radarbasierten Umfelddaten von mehreren Radarsensoren R kombinieren, um das Umfeld des Fahrzeuges F möglichst vollumfassend zu erkennen. Im Rahmen der Erfindung kann es von Vorteil sein, dass die radarbasierten Umfelddaten D3 mithilfe einer FMCW-Radartechnologie oder einer UWB-Radartechnologie erfasst und ausgewertet werden. Mithilfe von radarbasierten Umfelddaten können somit Vitalparameter VP einer Person P in der erkannten Notsituation N bestimmt werden.

Grundsätzlich kann das Verfahren durch ein Rettungsfahrzeug ausgeführt werden. Vorteilhafterweise kann ferner vorgesehen sein, dass, wenn sich das Rettungsfahrzeug einer Unfallstelle nähert, die optischen, akustischen und/oder radarbasierte Umfelddaten D1, D2, D3 und/oder Vitalparameter VP der Person P in der Notsituation N und/oder Klassifikationsdaten für die Notsituation N und/oder bestimmtes Gefährdungspotential den Rettungskräften, Notfallsanitätern und/oder Notfallärzten im Rettungsfahrzeug aufgezeigt und/oder zur Bewertung und/oder Veranlassung von weiteren Maßnahmen an einen Erste-Hilfe-Ort und/oder an eine Backendvorrichtung gesendet werden.

Das Verfahren kann aber auch durch private Fahrzeuge F ausgeführt werden.

Das Fahrzeug kann sich gegenüber der Person P in der Notsituation N entsprechend positionieren, um Vitalparameter VP der Person P mithilfe von Radarwellen auf eine verbesserte Weise bestimmen zu können.

Es ist ferner denkbar, dass, wenn eine Notsituation N erkannt wurde, ein spezielles Fahrzeug mit hochaufgelösten Umfeldsensoren geschickt wird (bspw. durch das Fahrzeug F selbst und/oder mithilfe einer Backendvorrichtung, die durch das Fahrzeug F über die Notsituation informiert wurde), um die Notsituation N möglichst genau zu analysieren und/oder mindestens eine weitere Hilfemaßnahme bereitzustellen.

Ein korrespondierendes Fahrzeug F stellt ebenfalls einen Aspekt der Erfindung dar.

Die voranstehende Erläuterung der Ausführungsformen beschreibt die vorliegende Erfindung ausschließlich im Rahmen von Beispielen. Selbstverständlich können einzelne Merkmale der Ausführungsformen, sofern technisch sinnvoll, frei miteinander kombiniert werden, ohne den Rahmen der vorliegenden Erfindung zu verlassen.

### Bezugszeichenliste

- D1: optische Umfelddaten
- D2: akustische Umfelddaten
- D3: radarbasierte Umfelddaten
- dt: Zeitraum
- ECU: Auswertevorrichtung
- F: Fahrzeug
- K: Kameravorrichtung
- M: Außenmikrofon
- N: Notsituation
- P: Person
- R: Radarsensor
- VP: Vitalparameter
- W: Wahrscheinlichkeit
- W1: erster Schwellenwert
- W2: zweiter Schwellenwert

## Patentansprüche

1. Verfahren zum Erkennen einer, insbesondere medizinischen, Notsituation (N) einer Person (P) in einem Umfeld eines automatisiert und/oder autonom fahrenden Fahrzeuges (F),
aufweisend:
- Erfassen von optischen und/oder akustischen Umfelddaten (D1, D2) für einen vorgegebenen Zeitraum (dt) durch mindestens eine Kameravorrichtung (K) und/oder mindestens ein Außenmikrofon (M) des Fahrzeuges (F),
- Auswerten von optischen und/oder akustischen Umfelddaten (D1, D2) durch eine Auswertevorrichtung (ECU) des Fahrzeuges (F), um eine Wahrscheinlichkeit (W) für die Notsituation (N) der Person (P) zu bestimmen, und insbesondere die Notsituation (N) zu klassifizieren, und vorzugsweise ein Gefährdungspotential für die Person (P) in der Notsituation (N), bevorzugt in Graden, zu bestimmen,
- Vergleichen der Wahrscheinlichkeit (W) mit einem ersten Schwellenwert (W1) und einem zweiten Schwellenwert (W2) durch die Auswertevorrichtung (ECU), wobei der zweite Schwellenwert (W2) größer als der erste Schwellenwert (W1) ist,
- Erkennen der Notsituation (N) der Person (P) durch die Auswertevorrichtung (ECU) in Abhängigkeit von dem Vergleichen.

2. Verfahren nach Anspruch 1,
**dadurch gekennzeichnet,**
**dass**, wenn die Wahrscheinlichkeit (W) für eine Notsituation (N) anhand von optischen und/oder akustischen Umfelddaten über dem ersten Schwellwert (W1) und unter dem zweiten Schwellenwert (W2) liegt, ferner aufweisend:
- Erfassen von radarbasierten Umfelddaten (D3) für den vorgegebenen Zeitraum (dt),
- Überprüfen, ob die radarbasierten Umfelddaten (D3) der Person (P) in der Notsituation (N) zugeordnet werden können,
- Auswerten von radarbasierten Umfelddaten (D3), die der Person in der Notsituation (N) zugeordnet werden können, um Vitalparameter (VP), bspw. Atem- und/ oder Herzfrequenz, der Person (P) in der erkannten Notsituation (N) zu bestimmen,
- Anpassen der Wahrscheinlichkeit (W) für die Notsituation (N) in Abhängigkeit von den Vitalparametern (VP),
- Vergleichen der angepassten Wahrscheinlichkeit (W) mit dem zweiten Schwellenwert (W2) und/oder
- Erkennen der Notsituation (N) mithilfe von radarbasierten Umfelddaten (D3), wenn die angepasste Wahrscheinlichkeit (W) über dem zweiten Schwellenwert (W2) liegt.

3. Verfahren nach Anspruch 1 oder 2,
**dadurch gekennzeichnet,**
**dass**, wenn die Wahrscheinlichkeit (W) für eine Notsituation (N) anhand von optischen und/oder akustischen Umfelddaten (D1, D2) über dem zweiten Schwellenwert (W2) liegt, ferner aufweisend:
- Erkennen der Notsituation (N) mithilfe von optischen und/oder akustischen Umfelddaten (D1, D2), insbesondere ohne radarbasierte Umfelddaten (D3) oder bevor radarbasiere Umfelddaten (D3) erfasst und ausgewertet werden.

4. Verfahren nach einem der vorhergehenden Ansprüche,
**dadurch gekennzeichnet,**
**dass**, wenn die Notsituation (N) erkannt wurde, mindestens eine Hilfemaßnahme durch die Auswertevorrichtung (ECU) veranlasst und/oder durchgeführt wird:
- Aussenden einer Notfallmeldung an Rettungskräfte, an einen Erste-Hilfe-Ort, an andere Fahrzeuge, an Infrastruktur und/oder an eine Backendvorrichtung,
- Anhalten des Fahrzeuges (F),
- Markieren und/oder Absichern eines Notfallortes,
- Bereitstellen einer Transportmöglichkeit für die Person (P) zu einem Erste-Hilfe-Ort,
- Übermitteln von Klassifikationsdaten für die Notsituation (N) an die Backendvorrichtung, an Rettungskräfte und/oder einen Erste-Hilfe-Ort,
- Übermitteln von Vitalparametern (VP) an die Backendvorrichtung, an Rettungskräfte und/oder einen Erste-Hilfe-Ort und/oder
- Übermitteln von optischen, akustischen und/oder radarbasierten Umfelddaten (D1, D2, D3) an die Backendvorrichtung, an Rettungskräfte und/oder an einen Erste-Hilfe-Ort.

5. Verfahren nach einem der vorhergehenden Ansprüche,
**dadurch gekennzeichnet,**
**dass** der vorgegebene Zeitraum (dt) und/oder die Schwellenwerte (W1, W2) in Abhängigkeit von mindestens einem von den folgenden Parametern bestimmt wird/werden:
- Ort,
- Verkehrssituation,
- Kontextsituation, z. B. erkannte Menschenansammlung unmittelbar bei der Person (P) in der Notsituation (N),
- Umgebungsbedingungen,
- Wetterbedingungen,
- Tageszeit,
- Jahreszeit und/oder
- Wochentag.

6. Verfahren nach einem der vorhergehenden Ansprüche,
**dadurch gekennzeichnet,**
**dass** die optischen Umfelddaten (D1) durch mindestens eine oder mehrere Außenkamera(s) erfasst werden.

7. Verfahren nach einem der vorhergehenden Ansprüche,
**dadurch gekennzeichnet,**
**dass** die optischen Umfelddaten (D1) mithilfe einer Bildverarbeitung und/oder einer Bildsegmentierung, insbesondere einer semantischen Bildsegmentierung, einer Objekterkennung, einer Gestenerkennung und/oder einer Bewegungserkennung ausgewertet werden.

8. Verfahren nach einem der vorhergehenden Ansprüche,
**dadurch gekennzeichnet,**
**dass** die akustischen Umfelddaten (D2) durch ein Stereosystem mit mindestens zwei oder mehreren Außenmikrofonen erfasst werden, um insbesondere eine Geräuschlokalisierung zu ermöglichen.

9. Verfahren nach einem der vorhergehenden Ansprüche,
**dadurch gekennzeichnet,**
**dass** die akustischen Umfelddaten (D2) mithilfe einer Geräuscherkennung, einer Geräuschlokalisierung und/oder einer Spracherkennung ausgewertet werden.

10. Verfahren nach einem der vorhergehenden Ansprüche,
**dadurch gekennzeichnet,**
**dass** die radarbasierten Umfelddaten (D3) durch ein Radarsystem mit mindestens zwei oder mehreren Radarsensoren (R) erfasst werden, um insbesondere eine hochaufgelöste Radaranalyse zu ermöglichen.

11. Verfahren nach einem der vorhergehenden Ansprüche,
**dadurch gekennzeichnet,**
**dass** die radarbasierten Umfelddaten (D3) mithilfe einer FMCW-Radartechnologie oder einer UWB-Radartechnologie erfasst und ausgewertet werden.

12. Verfahren nach einem der vorhergehenden Ansprüche,
**dadurch gekennzeichnet,**
**dass** das Verfahren durch ein Rettungsfahrzeug ausgeführt wird, wobei insbesondere wenn sich das Rettungsfahrzeug einer Unfallstelle nähert, die optischen, akustischen und/oder radarbasierte Umfelddaten (D1, D2, D3) und/oder Vitalparameter (VP) der Person (P) in der Notsituation (N) und/oder Klassifikationsdaten für die Notsituation (N) und/oder bestimmtes Gefährdungspotential den Rettungskräften, Notfallsanitätern und/oder Notfallärzten im Rettungsfahrzeug aufgezeigt und/oder zur Bewertung und/oder Veranlassung von weiteren Maßnahmen an einen Erste-Hilfe-Ort und/oder an eine Backendvorrichtung gesendet werden.

13. Verfahren nach einem der vorhergehenden Ansprüche,
**dadurch gekennzeichnet,**
**dass** das Fahrzeug zum Durchführen des Verfahrens sich gegenüber der Person (P) in der Notsituation (N) positioniert, um Vitalparameter (VP) der Person (P) auf eine verbesserte Weise bestimmen zu können.

14. Verfahren nach einem der vorhergehenden Ansprüche,
**dadurch gekennzeichnet,**
**dass**, wenn eine Notsituation (N) erkannt wurde, ein spezielles Fahrzeug mit hochaufgelösten Umfeldsensoren geschickt wird, um die Notsituation (N) zu verifizieren und/oder mindestens eine weitere Hilfemaßnahme bereitzustellen.

15. Fahrzeug zum Durchführen eines Verfahrens zum Erkennen einer, insbesondere medizinischen, Notsituation (N) einer Person (P) in einem Umfeld eines automatisiert und/oder autonom fahrenden Fahrzeuges (F) nach einem der vorhergehenden Ansprüche,
aufweisend:
- mindestens eine Kameravorrichtung (K) und/oder mindestens ein Außenmikrofon (M) und/oder mindestens einen Radarsensor (R) zum Erfassen von optischen, akustischen und/oder radarbasierten Umfelddaten (D1, D2, D3) für einen vorgegebenen Zeitraum (dt),
- eine Auswertevorrichtung (ECU) zum Auswerten von optischen, akustischen und/oder radarbasierten Umfelddaten (D1, D2, D3), um eine Wahrscheinlichkeit (W) für die Notsituation (N) der Person (P) zu bestimmen, und insbesondere die Notsituation (N) zu klassifizieren, und vorzugsweise ein Gefährdungspotential für die Person (P) in der Notsituation (N), bevorzugt in Graden, zu bestimmen,
wobei die Auswertevorrichtung (ECU) zum Vergleichen der Wahrscheinlichkeit (W) mit einem ersten Schwellenwert (W1) und einem zweiten Schwellenwert (W2) ausgeführt ist, wobei die Auswertevorrichtung (ECU) zum Erkennen der Notsituation (N) in Abhängigkeit von dem Vergleichen ausgeführt ist.
